(19) Europäisches Patentamt
European Patent Office
Office européen des brevets

(11) Publication number: **0 524 968 B1**

(12) **EUROPEAN PATENT SPECIFICATION**

(45) Date of publication of patent specification: **07.06.95**  (51) Int. Cl.[6]: **A61K 9/127**

(21) Application number: **91906733.0**

(22) Date of filing: **20.03.91**

(86) International application number:
**PCT/US91/01849**

(87) International publication number:
**WO 91/14445 (03.10.91 91/23)**

(54) HETEROVESICULAR LIPOSOMES.

(30) Priority: **21.03.90 US 496846**

(43) Date of publication of application:
**03.02.93 Bulletin 93/05**

(45) Publication of the grant of the patent:
**07.06.95 Bulletin 95/23**

(84) Designated Contracting States:
**AT BE CH DE DK ES FR GB GR IT LI LU NL SE**

(56) References cited:
**EP-A- 0 199 362**
**EP-A- 0 280 503**
**WO-A-85/00515**
**FR-A- 2 591 105**
**US-A- 4 235 871**

**Biochimica et Biophysica Acta, Vol. 728,
1983 (KIM), "Preparation ofMultivesicular
liposomes", pages 339-348 (See the entire
document).**

**CHEMICAL ABSTRACTS, vol. 107, No. 10, is-
sued 1987 (Columbus, Ohio, US),**

**KIM,"Multivesicular liposomes containing
cytarabine entrapped in the presence of-
hydrochloric acid for intracavity chemo-
therapy", see page 384, col. 1, theAbstract
No. 83830x.**

(73) Proprietor: **DEPOTECH CORPORATION**
**11025 North Torrey Pines Road,**
**Suite 100**
**La Jolla,**
**California 92837 (US)**

(72) Inventor: **KIM, Sinil**
**548 Ford Avenue**
**Solana Beach, CA 92705 (US)**

(74) Representative: **Wilkinson, Stephen John et al**
**Stevens, Hewlett & Perkins**
**1 St. Augustine's Place**
**Bristol BS1 4UD (GB)**

EP 0 524 968 B1

## Description

### Field of the Invention

The invention relates to the synthetic heterovesicular lipid vesicles or liposomes, processes for their manufacture and encapsulation of various materials therein, and treatment of patients with them.

### Background Art

Multivesicular liposomes are one of the three main types of liposomes, first made by Kim, et al. (1983, Biochim, Biophys. Acta 782, 339-348), and are uniquely different from the unilamellar (Huang, 1969, Biochemistry 8,344-352; Kim, et al. 1981, Biochim. Biophys. Acta 646, 1-10) and multilamellar (Bangham, et al. 1965, J. Mol, Bio. 13,238-252) liposomes in that there are multiple non-concentric aqueous chambers within. Previously described techniques for producing liposomes relate to the production of non-multivesicular liposomes; for example, U.S. Patent Nos. 4,522,803 - Lenk, 4,310,506 - Baldeschwieler, 4,235,871 - Papahadjopoulos, 4,224,179 - 4,078,052 - Papahadjopoulos, 4,394,372 - Taylor, 4,308,166 - Marchetti, 4,485,054 - Mezei, and 4,508,703 - Redziniak. For a comprehensive review of various methods of liposome preparation, refer to Szoka, et al. 1980, Ann. Rev. Biophys. Bioeng. 9:467-508.

Heterovesicular liposomes are lipid vesicles or liposomes with multiple internal aqueous chambers where at least two substances of different compositions are each encapsulated in separate chambers within one liposomes. The lipid vesicles or liposomes with multiple internal aqueous chambers include multilamellar liposomes, stable paucilamellar liposomes, and multivesicular liposomes. It is highly advantageous to provide a liposome delivery system in which two or more different substances are each encapsulated in separate compartments of a single liposome rather than encapsulated together in each compartment of the liposome.

### Summary of the Invention

The composition of the present invention comprises heterovesicular liposomes, i.e., lipid vesicles or liposomes with multiple internal aqueous chambers where two or more substances of different composition are each encapsulated separately in different chambers within one liposome.

Briefly, the method of the invention comprises making a "water-in-lipid" emulsion by dissolving amphipathic lipids in one or more organic solvents for the first lipid component, adding an immiscible first aqueous component including a substance to be encapsulated, preferably in the presence of hydrochloric acid, and then emulsifying the mixture mechanically. In the emulsion, the water droplets suspended in the organic solvent will form the internal aqueous chambers, and the monolayer of amphipathic lipids lining the aqueous chambers will become one leaflet of the bilayer membrane in the final product. A second lipid component is then formed by dissolving amphipathic lipids in a volatile organic solvent and adding an immiscible second aqueous component including a second substance to be encapsulated, preferably in the presence of hydrochloric acid. A second emulsion is then created. A chimeric emulsion is then formed by combining the first and second emulsions. The chimeric emulsion consists of multiple water droplets suspended in organic solvent where the substances of two different compositions are each dissolved separately in different aqueous droplets. The chimeric emulsion is then immersed in a third aqueous immiscible component preferably containing one or more nonionic osmotic agents and acid-neutralizing agent of low ionic strength and then mechanically dividing it to form solvent spherules suspended in the third aqueous component. The solvent spherules contain multiple aqueous droplets where the substances of two different compositions are each dissolved separately in different aqueous droplets within a single solvent spherule. The volatile organic solvent is evaporated from the spherules preferably by passing a stream of gas over the suspension. When the solvent is completely evaporated, the spherules convert into heterovesicular liposomes with multiple internal aqueous chambers where two substances of different compositions are encapsulated separately in different chambers within one liposome.

The use of hydrochloric acid with a neutralizing agent, or other hydrochlorides which slow leakage rates is preferably for high encapsulation efficiency and for a slow leakage rate of encapsulated molecules in biological fluids and in vivo. It is also preferable to use neutralizing agent of low ionic strength to prevent solvent spherules from sticking to each other.

Accordingly, it is an object of the present invention to provide a heterovesicular lipid vesicle or liposome having at least two substances of different compositions each encapsulated separately in different chambers of the vesicle or liposome.

2

A further object of the present invention is the provision of a heterovesicular liposome containing at least two biologically active substances of different compositions each encapsulated separately in chambers of the liposome in the presence of hydrochloric acid or other hydrochlorides which slow the leakage of them.

It is a further object of the present invention to provide a heterovesicular liposome containing at least two biologically active substances of different compositions each encapsulated separately in chambers of the liposome in the presence of hydrochloric acid or other hydrochlorides and a neutralizing agent.

It is a further object of the present invention to provide methods of producing such heterovesicular lipid vesicles or liposomes.

It is a further object of the present invention to provide processes for producing such heterovesicular lipid vesicles or liposomes by providing a first lipid component dissolved in one or more organic solvents and adding to the lipid component an immiscible first aqueous component containing a first substance to be encapsulated, forming a first water in oil emulsion from the first two immiscible components, providing a second lipid component dissolved in one or more organic solvents and adding into the lipid component an immiscible second aqueous component containing a second substance to be encapsulated, forming a second water in oil emulsion from the second two immiscible components, forming a chimeric emulsion by combining the first water in oil emulsion and second water in oil emulsion, transferring and immersing the chimeric emulsion into a third immiscible aqueous component, dispersing the chimeric emulsion to form solvent spherules containing multiple droplets of the first aqueous component containing the first substance and the second aqueous component containing the second substance, and evaporating the organic solvent from the solvent spherules to form the heterovesicular lipid vesicles or liposomes.

It is a further object to provide such a process in which a variety of hydrophilic biologically active materials can be encapsulated separately in chambers of the heterovesicular lipid vesicles or liposomes.

It is a further object of the present invention to provide a method for the treatment of a patient with at least two separate biologically active substances of different compositions by administering them to the patient encapsulated separately in chambers of a heterovesicular vesicle or liposome.

Other and further objects, features and advantages of the invention appear throughout the specification and claims.

## Brief Description of the Drawings

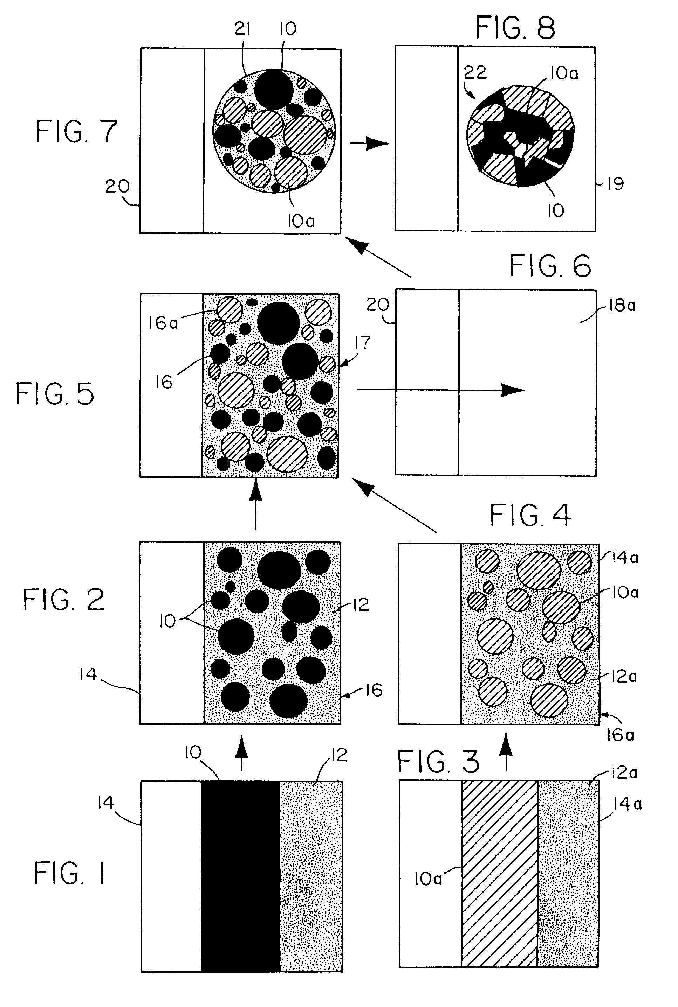

Figures 1-8 are schematic diagrams illustrating preparation of a heterovesicular vesicle or liposome.

## Description of Preferred Embodiments

The term "multivesicular liposomes" as used throughout the specification and claims means man-made, microscopic lipid-vesicles consisting of lipid bilayer membranes, enclosing multiple non-concentric aqueous chambers which all contain the same component. In contrast, the term "heterovesicular liposomes" as used throughout the specification and claims means man-made, microscopic liquid vesicles consisting of lipid bilayer membranes enclosing multiple, aqueous chambers wherein at least two of the chambers separately contain substances of different compositions. The microscopic lipid vesicles include multilamellar liposomes, stable paucilamellar liposomes, and multivesicular liposomes.

The term "chimeric emulsion" as used throughout the specification and claims means an emulsion that consists of multiple water droplets suspended in organic solvent where the substances of two different compositions are each dissolved separately in different aqueous droplets.

The term "solvent spherule" as used throughout the specification and claims means a microscopic spheroid droplet of organic solvent, within which is multiple smaller droplets of aqueous solution. The solvent spherules are suspended and totally immersed in a second aqueous solution.

The term "neutral lipid" means oil or fats that have no membrane-forming capability by themselves and lack a hydrophilic "head" group.

The term amphipathic lipids means those molecules that have a hydrophilic "head" group and hydrophobic "tail" group and have membrane-forming capability.

The composition of the present invention is a heterovesicular lipid vesicle or liposome having at least two substances of different compositions each encapsulated separately in different chambers of the vesicle or liposome.

Many and varied biological substances can be incorporated by encapsulation within the multivesicular liposomes. These include drugs, and other kinds of materials, such as DNA, RNA, proteins of various types, protein hormones produced by recombinant DNA technology effective in humans, hematopoietic growth factors, monokines, lymphokines, tumor necrosis factor, inhibin, tumor growth factor alpha and beta, mullerian inhibitory substance, nerve growth factor, fibroblast growth factor, platelet-derived growth factor, pituitary and hypophyseal hormones including LH and other releasing hormones, calcitonin, proteins that serve as immunogens for vaccination, and DNA and RNA sequences.

The following Table 1 includes a list of representative biologically active substances which can be encapsulated in heterovesicular liposomes in the presence of a hydrochloride and which are effective in humans.

TABLE 1

| Antiasthma | Antiarrhythmic | Tranquilizers |
|---|---|---|
| metaproterenol | propanolol | chlorpromazine |
| aminophylline | atenolol | benzodiazepine |
| theophylline | verapamil | butyrophenones |
| terbutaline | captopril | hydroxyzines |
| Tegretol | isosorbide | meprobamate |
| ephedrine | | phenothiazines |
| isoproterenol | | reserpine |
| adrenalin | | thioxanthines |
| norepinephrine | | |

| Cardiac glycosides | Hormones | Steroids |
|---|---|---|
| digitalis | antidiuretic | prednisone |
| digitoxin | corticosteroids | triamcinolone |
| lanatoside C | testosterone | hydrocortisone |
| digoxin | estrogen | dexamethasone |
| | thyroid | betamethosone |
| | growth | prednisolone |
| | ACTH | |
| | progesterone | |
| | gonadotropin | |
| | mineralocorticoid | |
| | LH | |
| | LHRH | |
| | FSH | |
| | calcitonin | |

| Antihypertensives | Antidiabetic | Antihistamines |
|---|---|---|
| apresoline | Diabenese | pyribenzamine |
| atenolol | insulin | chlorpheniramine |
| | | diphenhydramine |

| Antiparasitic | Anticancer | Sedatives & Analgesic |
|---|---|---|
| praziquantel | azathioprine | morphine |
| metronidazole | bleomycin | dilaudid |
| pentamidine | cyclophosphamide | codeine |
| | adriamycin | codeine-like synthetics |
| | daunorubicin | demerol |
| | vincristine | oxymorphone |
| | methotrexate | phenobarbital |
| | 6-TG | barbiturates |
| | 6-MP | |
| | vinblastine | |
| | VP-16 | |
| | VM-26 | |
| | cisplatine | |
| | FU | |

| Antibiotic | Immunoptherapies | Vaccines |
|---|---|---|
| penicillin | interferon | influenza |
| tetracycline | interleukin-2 | respiratory syncytial |
| erythromycin | monoclonal antibodies | virus |
| cephalothin | gammaglobulin | Hemophilus influenza |
| imipenem | | vaccine |
| cefofaxime | | |
| carbenicillin | | |

| Antibiotic (continued) | Antifungal | Antiviral |
|---|---|---|
| vancomycin | amphotericin B | acyclovir and deriva- |
| gentamycin | myconazole |     tives |
| tobramycin | muramyl dipeptide | Winthrop-51711 |
| piperacillin | clotrimazole | ribavirin |
| moxalactam | | rimantadine/amantadine |
| amoxicillin | | azidothymidine & deriva- |
| ampicillin | Antihypotension |     tives |
| cefazolin | dopamine | adenine arabinoside |
| cefadroxil | dextroamphetamine | amidine-type protease |
| cefoxitin | |     inhibitors |
| other aminoglycosides | | |

| Proteins and Glycoproteins | Other |
|---|---|
| lymphokines | cell surface receptor |
|     interleukins - 1, 2, 3, 4, 5, and 6 |     blockers |
| cytokines | |
|     GM-CSF | Nucleic Acids & Analogs |
|     M-CSF | DNA |
|     G-CSF | RNA |
| tumor necrosis factor | methylphosphonates |
| inhibin |     and analogs |
| tumor growth factor | |
| Mullerian inhibitors substance | |
| nerve growth factor | |
| fibroblast growth factor | |
| platelet derived growth factor | |
| coagulation factors (e.g. VIII, IX, VII) | |
| insulin | |
| tissue plasminogen activator | |
| histocompatibility antigen | |
| oncogene products | |
| myelin basic protein | |
| collagen | |
| fibronectin | |
| laminin | |
| other proteins made by recombinant DNA | |
|     technology | |

A preferred method of making the heterovesicular vesicle or liposome is illustrated in the drawing to which reference is now made. In step 1 (Figure 1) a first aqueous substance of composition 10 to be encapsulated is added to a first lipid component 12 in the vial 14. The vial 14 is sealed and in step 2 (Figure 2) is mixed and shaken, such as being attached to the head of a vortex mixer to form the first water in oil emulsion 16 containing the first substance of composition 10 to be encapsulated. In step 3 (Figure 3), a second vial 14a, a second aqueous substance 10a to be encapsulated is added to a second lipid component 12a, and the vial 14a is sealed and in step 4 (Figure 4) is mixed, such as being attached to the head of a vortex mixer to form a second water-in-oil emulsion 16a containing the substance of composition 10a to be encapsulated.

6

EP 0 524 968 B1

In step 5 (Figure 5) the first 16 and second 16a water in oil emulsions are added together and mixed, such as by hand to make a "chimeric" emulsion 17.

In step 6 (Figure 6) a portion of the chimeric emulsion from step 5 is individually added to vials containing a third immiscible aqueous component 18a such as by squirting rapidly through a narrow tip pasteur pipette into two one-dram vials 20, here shown as one.

In step 7 (Figure 7) vials from step 6 are shaken, such as by a vortex mixer to form a chloroform spherule suspension 21, and in step 8 (Figure 8) the chloroform spherule suspension 21 in each vial 18a is transferred into an evaporation tank 19 from step 7 and the chloroform is evaporated, such as by a stream of nitrogen gas, thereby providing the heterovesicular liposome 22 that contains a first substance 10 in one or more internal aqueous chambers and a second substance 10a in the remaining internal aqueous chambers within a single liposome 22.

Preferably, each of the substances to be encapsulated are encapsulated in the presence of a hydrochloride, such as hydrochloric acid, which slows their leakage rate from the liposome or vesicle.

As previously mentioned, any biologically active substance, such as illustrated in Table 1, can be encapsulated separately in chambers of the vesicle or liposome.

The following examples set forth presently preferred methods of encapsulating two substances of different compositions in separate chambers of a vesicle or liposome.

Example 1

Preparation of Dideoxycytidine/Glucose Heterovesicular Liposomes

Step 1: A first aqueous substance (one ml of 20 mg/ml dideoxycytidine solution in water with 0.1 N hydrochloric acid) was added into a one-dram vial containing the first lipid component (9.3 $\mu$moles of dioleoyl lecithin, 2.1 $\mu$moles of dipalmitoyl phosphatidylglycerol, 15 $\mu$moles of cholesterol, 1.8 $\mu$moles of triolein and one ml of chloroform).

Step 2: The first vial was sealed and attached to the head of a vortex mixer and shaken at maximum speed for 6 minutes to form the first water-in-oil emulsion.

Step 3: In second vial, the second aqueous substance (one ml of 30 mg/ml glucose solution in water with 0.1 N hydrochloric acid) was added into the second lipid component (which is identical to the first lipid component).

Step 4: The second vial was sealed and attached to the head of a vortex mixer and shaken at maximum speed for 6 minutes to form the second water-in-oil emulsion.

Step 5: 0.5 ml of the first emulsion was added to the second vial and mixed by hand to make a "chimeric" emulsion.

Step 6: Half of the "chimeric" emulsion was individually squirted rapidly through a narrow tip Pasteur pipette into one-dram vials, each containing a third immiscible aqueous component (2.5 ml water, 32 mg/ml glucose, 40 mM free-base lysine.

Step 7: The vials from step 6 were shaken on the vortex mixer for 3 seconds at "5" setting to form solvent spherules containing multiple droplets of the first and second aqueous substances within.

Step 8: The chloroform spherule suspensions in each vials were transferred into the bottom of a 2 L beaker containing 4.5 ml of water, 35 mg/ml glucose, and 22 mM free-base lysine. A stream of nitrogen gas at 7 L/min was flushed through the beaker to evaporate chloroform over 5 minutes at 15 deg. C.

The above example describes a method of making heterovesicular liposomes which separately contain glucose in approximately 5/6 of the internal aqueous chambers and separately contain dideoxycytidine in the remaining 1/6 of the internal aqueous chambers within a single liposome. Heterovesicular liposomes containing dideoxycytidine solution as one aqueous substance and glucose as the second aqueous substance were markedly more stable than non-heterovesicular liposomes.

Example 2

This example is for the synthesis of heterovesicular liposomes containing IL-2 (interleukin-2) and lysine hydrochloride: For each batch of liposomes prepared, one ml of water containing 10 mg/ml HSA (Human serum albumin), 1 $\mu$g of IL-2, 200 mM lysine HCl pH 7.13 was added into a one-dram vial containing 9.3 $\mu$moles of dioleoyl lecithin, 2.1 $\mu$moles of dipalmitoyl phosphatidylglycerol, 15 $\mu$moles of cholesterol, and 1.8 $\mu$moles of triolein and one ml of chloroform (this is the first water-in-oil emulsion). For the second water-in-oil emulsion, 1 ml of lysine HCl (without IL-2) was added into one-dram vial containing 9.3 $\mu$moles of dioleoyl lecithin, 2.1 $\mu$moles of dipalmitoyl phosphatidylglycerol, 15 $\mu$moles of cholesterol, and 1.87 $\mu$moles

7

of triolein and one ml of chloroform. Each of the two vials were individually attached to the head of a vortex mixer and shaken sequentially at the maximum speed for 6 minutes.

0.5 ml of the first water-in-oil emulsion was added to the 2 ml of the second emulsion and mixed to make a "chimeric" water-in-oil emulsion. Half of the "chimeric" emulsion was individually squirted rapidly through a narrow tip Pasteur pipette into one-dram vials, each containing 2.5 ml of 4% glucose in water and 0.1 ml of lysine free base, 200 mM, and shaken at maximum speed for 3 seconds to form chloroform spherules. The chloroform spherule suspensions were transferred into 250 ml Erlenmeyer flask containing 5 ml of 4% glucose in water and 0.2 ml of lysine free base, 200 mM. A stream of nitrogen gas at 7 L/min was flushed through the flask to evaporate chloroform over 5 minutes at 37 degrees C.

Example 3

This example is for the synthesis of heterovesicular liposomes containing ara-C solution as the first aqueous substance and distilled water as the second aqueous substance. For each batch of liposomes prepared, one ml of water containing 100 mg/ml ara-C, pH 1.1 was added into a one-dram vial containing 9.3 $\mu$moles of dioleoyl lecithin, 2.1 $\mu$moles of dipalmitoyl phosphatidylglycerol, 15 $\mu$moles of cholesterol, and 1.8 $\mu$moles of triolein and one ml of chloroform, attached to the head of the vortex mixer and shaken at maximum speed for 6 minutes (this is the first water-in-oil emulsion). For the in situ generation of the second water-in-oil emulsion, 1/2 of the content was removed from the first water-in-oil emulsion, and then 1 ml of distilled water was added into the remaining first water-in-oil emulsion and the one-dram vial was shaken for 10 seconds at maximum speed. This resulted in a "chimeric" water-in-oil emulsion. Half of the "chimeric" emulsion was individually squired rapidly through a narrow tip Pasteur pipette into one-dram vials, each containing 2.0 ml of 4% glucose in water and 0.5 ml of lysine free base, 200 mM, and shaken at maximum speed for 3 seconds to form chloroform spherules. The chloroform spherule suspensions were transferred into 250 ml Erlenmeyer flask containing 4 ml of 4% glucose in water and 0.5 ml of lysine free base, 200 mM. A stream of nitrogen gas at 7 L/min was flushed through the flask to evaporate chloroform over 5 minutes at 37 degrees C.

Example 4

Synthesis of Heterovesicular Liposomes Containing Granulocyte-Macrophase Colony Stimulating Factor (GM-CSF)

Exactly the same procedure was used as in Example 2 except IL-2 was replaced with 1 $\mu$g of GM-CSF.

Example 5

Synthesis of Heterovesicular Liposomes of Various Lipid Composition, and Incorporation of Various Materials into Liposomes

In place of using dioleoyl lecithin, dipalmtoyl phosphatidylglyerol, cholesterol, and triolein (TO), and other amphipathic lipids such as phosphatidyl cholines (PC), cardiolipin (CL), dimyristoyl phosphatidylglycerol (DMPG), phosphatidyl ethanolamines (PE), phosphatidyl serines (PS), dimyristoyl phosphatidic acid (DMPA), and other neutral lipids such as tricaprylin (TC) in various combination can be used with similar results. For example, PC/C/CL/TO in 4.5/4.5/1/1 molar ration; DOPC/C/PS/TO in 4.5/4.5/1/1 molar ratio; PC/C/DPPG/TC in 5/4/1/1 molar ratio; PC/C/PG/TC in 5/4/1/1 molar ratio; PE/C/CL/TO in 4.5/4.5/1/1 molar ratio; and PC/C/DMPA/TO in 4.5/4.5/1/1 molar ratio can all be used. To incorporate other water-soluble materials, such as glucose, sucrose, methotrexate, Ponceau S, simply substitute the desired materials for IL-2 in Example 2. Also, other biologically active substances, such as set forth in Table 1, in suitable doses can be similarly substituted for IL-2 as in Example 2.

Example 6

In this example, the triolein in lipid components of above examples are substituted either singly or in combination by other triglycerides, vegetable oils, animal fats, tocopherols, tocopherol esters, cholesteryl esters, or hydrocarbons with good results.

Example 7

To make liposomes smaller than that in the foregoing examples, and with reference to Examples 1 or 2, the mechanical strength or duration of shaking or homogenization in Step 4 of Example 1 or 2 was increased. To make liposomes larger, the mechanical strength or duration of shaking or homogenisation in Step 4 of Example 1 or 2 was decreased.

The heterovesicular liposomes can be administered to the patients in the normal manner when it is desirable to provide two separate biologically active compounds to the patient for the particular purpose of treatment desired.

The dosage range appropriate for human use includes the range of 1-6000 mg/m$^2$ to body surface area. The reason that this range is so large is that for some applications, such as subcutaneous administration, the dose required may be quite small, but for other applications, such as intraperitoneal administration, the dose required to be used may be absolutely enormous. While doses outside the foregoing dose range may be given, this range encompasses the breadth of use for practically all the biologically active substances.

The multivesicular liposomes may be administered by any desired route; for example, intrathecal, intraperitoneal, subcutaneous, intravenous, intralymphatic, oral and submucosal, under many different kinds of epithelia including the bronchialar epithelia, the gastrointestinal epithelia, the urogenital epithelia, and various mucous membranes of the body, and intramuscular.

When encapsulating more than two substances separately in chambers of a liposome, a third (or fourth) aqueous component containing the third or fourth biologically active substance is formed, mixed to form a third or fourth water in oil emulsion, and then combined with the first and second emulsions and mixed to form a "chimeric" emulsion containing the three or more biologically active substances. The remainder of the process is the same as described when encapsulating two biologically active compounds or substances.

The present invention, therefore, obtains the objects and ends and has the advantages mentioned as well as others inherent therein.

While examples of the invention have been given for the purpose of disclosure, changes can be made therein which are within the spirit of the invention as defined by the appended claims.

**Claims**

1. A heterovesicular lipid vesicle or liposome having at least two different biologically active substances encapsulated in separate chambers of the same liposome.

2. A heterovesicular liposome as claimed in claim 1, wherein at least one of the biologically active substances is encapsulated in the presence of a hydrochloride.

3. A heterovesicular liposome as claimed in claim 2, wherein the hydrochloride is selected from the group consisting of hydrochloric acid, lysine hydrochloride, histidine hydrochloride and combinations thereof.

4. A heterovesicular liposome as claimed in any one of claims 1 to 3, wherein at least one of the biologically active substances is encapsulated in the presence of hydrochloric acid or other acid hydrochlorides and a neutralizing agent.

5. A heterovesicular liposome as claimed in any one of claims 1 to 4, wherein the biologically active substances are selected from the compositions of Table 1.

6. A heterovesicular lipid vesicle or liposome as claimed in any one of claims 1 to 4, wherein the biologically active substances are selected from the group consisting of antiarrhythmic, antiasthma, antibiotic, anticancer, antidiabetic, antifungal, antihistamines, antihypertensives, antihypotension, antiparasitic, antiviral, cell surface receptor blockers, glucose, cardiac glycosides, hormones, immunoptherapies, nucleic acids and analogs, proteins and glycoproteins, sedatives and analgesic, steroids, tranquilizers, vaccines and water.

7. A process for producing a heterovesicular lipid vesicle or liposome having at least two different biologically active substances separately encapsulated in aqueous chambers thereof comprising the steps of:

(a) providing a first lipid component dissolved in one or more organic solvents and adding into the said lipid component an immiscible first aqueous component containing a first biologically active substance to be encapsulated;

(b) forming a first water-in-oil emulsion from the first two immiscible components;

(c) providing a second lipid component dissolved in one or more organic solvents and adding into the said lipid component an immiscible second aqueous component containing a second substance to be encapsulated;

(d) forming a second water-in-oil emulsion from the second two immiscible components;

(e) forming a chimeric emulsion by combining the first water-in-oil emulsion and the second water-in-oil emulsion;

(f) transferring and immersing the product of step (e) in a third media that is immiscible with said organic solvents;

(g) dispersing the chimeric emulsion to form solvent spherules containing multiple droplets of the first aqueous component containing the first substance and the second aqueous component containing the second substance; and

(h) evaporating the organic solvents from the solvent spherules to form the heterovesicular liposomes.

8. The process according to Claim 7 wherein three or more water-in-oil emulsions containing three or more immiscible aqueous components are combined to form the chimeric emulsion.

9. The process according to Claim 7 or Claim 8 wherein the first and second lipid components are identical.

10. The process according to any one of Claims 7 to 9 wherein one or more of the lipid components contain a lipid with a net negative charge or charges.

11. The process according to any one of Claims 7 to 9 wherein at least one of the first and second lipid components is a neutral lipid either singly or in combination with a substance selected from the group consisting of triglycerides, vegetable oils, animal fats, tocopherols, tocopherol esters, cholesteryl esters and hydrocarbons.

12. The process according to any one of Claims 7 to 11 wherein the first and second lipid components are a phospholipid or an admixture of several phospholipids.

13. The process according to Claim 12 wherein at least one of the phospholipids is provided in admixture with cholesterol.

14. The process according to Claim 12 or Claim 13 wherein at least one of the phospholipids is provided in admixture with stearylamine.

15. The process according to any one of Claims 12 to 14 wherein the phospholipids are selected from the group consisting of phosphatidylcholine, cardiolipin, phosphatidylethanolamine, sphingomyelin, lysophosphatidylcholine, phosphatidylserine, phosphatidylinositol, phosphatidylglycerol and phosphatidic acid.

16. The process according to any one of Claims 7 to 15 wherein at least one of the first and second substances is a lipophilic biologically active material.

17. The process according to any one of Claims 7 to 15 wherein the biologically active substance is hydrophilic.

18. The process according to Claim 17 wherein the hydrophilic biologically active substance is selected from the group consisting of interleukin-2, cytosine arabinoside, methotrexate, 5-fluorouracil, cisplatin, floxuridine, melphalan, mercaptopurine, thioguanine, thiotepa, vincristine, vinblastine, streptozocin, leuprolide, interferon, calcitonin, doxorubicin, daunorubicin, mitoxanthrone, amacrine, actinomycin and bleomycin.

**19.** The process according to any one of Claims 7 to 15 where the biologically active substances are selected from the group consisting of antiarrhythmic, antiasthma, antibiotic, anticancer, antidiabetic, antifungal, antihistamines, antihypertensives, antihypotension, antiparasitic, antiviral, cell surface receptor blockers, glucose, cardiac glycosides, hormones, immunoptherapies, nucleic acids and analogs, proteins and glycoproteins, sedatives and analgesic, steroids, tranquilizers, vaccines and water.

**20.** The process according to any one of Claims 7 to 15 wherein the biologically active substances to be encapsulated are selected from the group consisting of the compositions of Table 1.

**21.** The process according to any one of Claims 7 to 20, wherein the organic solvent is selected from the group consisting of ethers, hydrocarbons, halogenated hydrocarbons, halogenated ethers, esters and combinations thereof.

**22.** The process according to any one of Claims 7 to 21, wherein at least one of the biologically active substances is encapsulated in the presence of a hydrochloride.

**23.** The process according to Claim 22, wherein the hydrochloride is selected from the group consisting of hydrochloric acid, lysine hydrochloride, histidine hydrochloride and combinations thereof.

**24.** The process according to any one of claims 7 to 23, wherein the emulsification of the two components is carried out using methods selected from the group consisting of mechanical agitation, ultrasonic energy, and nozzle atomization.

**25.** The process according to any one of Claims 7 to 24, wherein the third aqueous component contains at least one acid-neutralizing agent.

**26.** The process according to Claim 25 wherein the acid-neutralizing agent is selected either singly or in combination from the group consisting of free-base lysine and free-base histidine.

**27.** The process according to Claim 25 or Claim 26 wherein the third aqueous component is an aqueous solution further containing solutes selected from the group consisting of carbohydrates and amino acids.

**28.** The process according to Claim 27 wherein the solutes are selected either singly or in combination from the group consisting of glucose, sucrose, lactose, free-base lysine and free-base histidine.

**29.** The process according to any one of Claims 7 to 28 wherein the dispersion to form solvent spherules is carried out using methods selected from the group consisting of mechanical agitation, ultrasonic energy and nozzle atomization.

**30.** The process according to any one of Claims 7 to 29 wherein the evaporation of the organic solvent is provided by passing nitrogen gas over the second aqueous component.

**Patentansprüche**

**1.** Heterovesikuläres Lipidvesikel oder Liposom mit mindestens zwei unterschiedlichen biologisch aktiven Substanzen, die in getrennten Kammern des gleichen Liposoms eingekapselt sind.

**2.** Heterovesikuläres Liposom nach Anspruch 1, wobei mindestens eine der biologisch aktiven Substanzen in Gegenwart eines Hydrochlorids eingekapselt ist.

**3.** Heterovesikuläres Liposom nach Anspruch 2, wobei das Hydrochlorid ausgewählt ist aus Salzsäure, Lysinhydrochlorid, Histidinhydrochlorid und Kombinationen davon.

**4.** Heterovesikuläres Liposom nach einem der Ansprüche 1 bis 3, wobei mindestens eine der biologisch aktiven Substanzen in Gegenwart von Salzsäure oder anderen sauren Hydrochloriden und einem neutralisierenden Wirkstoff eingekapselt ist.

5. Heterovesikuläres Liposom nach einem der Ansprüche 1 bis 4, wobei die biologisch aktiven Substanzen ausgewählt sind aus den Zusammensetzungen von Tabelle 1.

6. Heterovesikuläres Lipidvesikel oder Liposom nach einem der Ansprüche 1 bis 4, wobei die biologisch aktiven Substanzen ausgewählt sind aus antiarrhythmischen Substanzen, antiasthmatischen Substanzen, Antibiotika, Antikrebsmitteln, Mitteln gegen Diabetes, Antipilzmitteln, Antihistaminika, Antihypertonika, Antihypotonika, gegen Parasiten wirkenden Mitteln, antiviralen Mitteln, Zelloberflächenrezeptorblokkern, Glucose, Herzglycosiden, Hormonen, Mitteln für Immuntherapie, Nucleinsäuren und Analogen, Proteinen und Glycoproteinen, Sedativa und Analgetika, Steroiden, Tranquilizern, Impfstoffen und Wasser.

7. Verfahren zur Herstellung eines heterovesikulären Lipidvesikels oder Liposoms mit mindestens zwei unterschiedlichen biologisch aktiven Substanzen, die getrennt in wäßrigen Kammern davon eingekapselt sind, die folgenden Schritte umfassend:
    (a) Bereitstellen eines ersten Lipidbestandteils, der in einem oder mehreren organischen Lösungsmitteln gelöst ist, und Zugabe eines ersten unvermischbaren wäßrigen Bestandteils, der eine erste einzukapselnde biologisch aktive Substanz enthält, zu dem Lipidbestandteil;
    (b) Bildung einer ersten Wasser-in-Öl-Emulsion aus den ersten zwei unvermischbaren Bestandteilen;
    (c) Bereitstellen eines zweiten Lipidbestandteils, der in einem oder mehreren organischen Lösungsmitteln gelöst ist, und Zugabe eines zweiten unvermischbaren Bestandteils, der eine zweite einzukapselnde Substanz enthält, zu dem Lipidbestandteil;
    (d) Bildung einer zweiten Wasser-in-Öl-Emulsion aus den zweiten zwei unvermischbaren Bestandteilen;
    (e) Bildung einer chimären Emulsion durch Kombinieren der ersten Wasser-in-Öl-Emulsion mit der zweiten Wasser-in-Öl-Emulsion;
    (f) Überführen und Eintauchen des Produkts aus Schritt (e) in ein drittes Medium, das mit den organischen Lösungsmitteln unvermischbar ist;
    (g) Dispergieren der chimären Emulsion zur Bildung von Lösungsmittelkügelchen, die mehrere Tröpfchen des ersten wäßrigen Bestandteils enthalten, der die erste Substanz enthält, und des zweiten wäßrigen Bestandteils, der die zweite Substanz enthält; und
    (h) Verdampfen der organischen Lösungsmittel aus den Lösungsmittelkügelchen zur Bildung der heterovesikulären Liposomen.

8. Verfahren nach Anspruch 7, wobei drei oder mehr Wasser-in-Öl-Emulsionen, die drei oder mehr unvermischbare wäßrige Bestandteile enthalten, zum Erhalt einer chimären Emulsion kombiniert werden.

9. Verfahren nach Anspruch 7 oder 8, wobei die ersten und zweiten Lipidbestandteile identisch sind.

10. Verfahren nach einem der Ansprüche 7 bis 9, wobei einer oder mehrere der Lipidbestandteile ein Lipid mit einer negativen Gesamtladung oder -Ladungen enthalten.

11. Verfahren nach einem der Ansprüche 7 bis 9, wobei mindestens einer der ersten und zweiten Lipidbestandteile ein neutrales Lipid ist, entweder einzeln oder in Kombination mit einer Substanz, die ausgewählt ist aus Triglyceriden, Pflanzenölen, tierischen Fetten, Tocopherolen, Tocopherolestern, Cholesterylestern und Kohlenwasserstoffen.

12. Verfahren nach einem der Ansprüche 7 bis 11, wobei die ersten und zweiten Lipidbestandteile ein Phospholipid oder ein Gemisch von verschiedenen Phospholipiden sind.

13. Verfahren nach Anspruch 12, wobei mindestens eines der Phospholipide als Gemisch mit Cholesterin bereitgestellt wird.

14. Verfahren nach Anspruch 12 oder 13, wobei mindestens eines der Phospholipide als Gemisch mit Stearylamin bereitgestellt wird.

15. Verfahren nach einem der Ansprüche 12 bis 14, wobei die Phospholipide ausgewählt sind aus Phosphatidylcholin, Cardiolipin, Phosphatidylethanolamin, Sphingomyelin, Lysophosphatidylcholin, Phosphatidylserin, Phosphatidylinosit, Phosphatidylglycerin und Phosphatidsäure.

**16.** Verfahren nach einem der Ansprüche 7 bis 15, wobei mindestens eine der ersten und zweiten Substanzen ein lipophiles, biologisch aktives Material ist.

**17.** Verfahren nach einem der Ansprüche 7 bis 15, wobei die biologisch aktive Substanz hydrophil ist.

**18.** Verfahren nach Anspruch 17, wobei die hydrophile biologisch aktive Substanz ausgewählt ist aus Interleukin-2, Cytosinarabinosid, Methotrexat, 5-Fluoruracil, Cisplatin, Floxuridin, Melphalan, Mercaptopurin, Thioguanin, Thiotepa, Vincristin, Vinblastin, Streptozocin, Leuprolid, Interferon, Calcitonin, Doxorubicin, Daunorubicin, Mitoxanthron, Amacrin, Actinomycin und Bleomycin.

**19.** Verfahren nach einem der Ansprüche 7 bis 15, wobei die biologisch aktiven Substanzen ausgewählt sind aus antiarrhythmischen Substanzen, antiasthmatischen Substanzen, Antibiotika, Antikrebsmitteln, Mitteln gegen Diabetes, Antipilzmitteln, Antihistaminika, Antihypertonika, Antihypotonika, gegen Parasiten wirkenden Mitteln, antiviralen Mitteln, Zelloberflächenrezeptorblockern, Glucose, Herzglycosiden, Hormonen, Mitteln für Immuntherapie, Nucleinsäuren und Analogen, Proteinen und Glycoproteinen, Sedativa und Analgetika, Steroiden, Tranquilizern, Impfstoffen und Wasser.

**20.** Verfahren nach einem der Ansprüche 7 bis 15, wobei die biologisch aktiven einzukapselnden Substanzen ausgewählt sind aus der Gruppe, die aus den Zusammensetzungen von Tabelle 1 besteht.

**21.** Verfahren nach einem der Ansprüche 7 bis 20, wobei das organische Lösungsmittel ausgewählt ist aus Ethern, Kohlenwasserstoffen, halogenierten Kohlenwasserstoffen, halogenierten Ethern, Estern und Kombinationen davon.

**22.** Verfahren nach einem der Ansprüche 7 bis 21, wobei mindestens eine der biologisch aktiven Substanzen in Gegenwart eines Hydrochlorids eingekapselt wird.

**23.** Verfahren nach Anspruch 22, wobei das Hydrochlorid ausgewählt ist aus Salzsäure, Lysinhydrochlorid, Histidinhydrochlorid und Kombinationen davon.

**24.** Verfahren nach einem der Ansprüche 7 bis 23, wobei das Emulgieren der zwei Bestandteile unter Verwendung von Verfahren durchgeführt wird, die ausgewählt sind aus mechanischem Rühren, Ultraschallbehandlung und Düsenversprühung.

**25.** Verfahren nach einem der Ansprüche 7 bis 24, wobei der dritte wäßrige Bestandteil mindestens einen Säure-neutralisierenden Wirkstoff enthält.

**26.** Verfahren nach Anspruch 25, wobei der Säure-neutralisierende Wirkstoff entweder einzeln oder in Kombination aus der Gruppe Lysin als freie Base und Histidin als freie Base ausgewählt ist.

**27.** Verfahren nach Anspruch 25 oder 26, wobei der dritte wäßrige Bestandteil eine wäßrige Lösung ist, die außerdem gelöste Stoffe enthält, ausgewählt aus Kohlenwasserstoffen und Aminosäuren.

**28.** Verfahren nach Anspruch 27, wobei die gelösten Stoffe entweder einzeln oder in Kombination aus der Gruppe Glucose, Saccharose, Lactose, Lysin als freie Base und Histidin als freie Base ausgewählt sind.

**29.** Verfahren nach einem der Ansprüche 7 bis 28, wobei das Dispergieren zum Erhalt von Lösungsmittelkügelchen unter Verwendung von Verfahren durchgeführt wird, die ausgewählt sind aus mechanischem Rühren, Ultraschallbehandlung und Düsenversprühung.

**30.** Verfahren nach einem der Ansprüche 7 bis 29, wobei die Verdampfung des organischen Lösungsmittels dadurch erfolgt, daß man Stickstoffgas über den zweiten wäßrigen Bestandteil leitet.

**Revendications**

1. Vésicule ou liposome lipidique hétérovésiculaire possédant au moins deux substances différentes biologiquement actives, encapsulées dans des cavités séparées du même liposome.

2. Liposome hétérovasculaire selon la revendication 1, dans lequel au moins l'une des substances biologiquement actives est encapsulée en présence d'un chlorhydrate.

3. Liposome hétérovasculaire selon la revendication 2, dans lequel on choisit le chlorhydrate dans le groupe constitué de l'acide chlorhydrique, du chlorhydrate de lysine, du chlorhydrate d'histidine et de leurs associations.

4. Liposome hétérovasculaire selon l'une quelconque des revendications 1 à 3, dans lequel au moins l'une des substances biologiquement actives est encapsulée en présence d'acide chlorhydrique et d'autres chlorhydrates d'acides, ainsi que d'un agent neutralisant.

5. Liposome hétérovasculaire selon l'une quelconque des revendications 1 à 4, dans lequel on choisit les substances biologiquement actives parmi les compositions du tableau 1.

6. Vésicule ou liposome lipidique hétérovasculaire selon l'une quelconque des revendications 1 à 4, dans laquelle ou lequel on choisit les substances biologiquement actives dans le groupe constitué d'un antiarythmique, d'un antiasthmatique, d'un antibiotique, d'un anticancéreux, d'un antidiabétique, d'un antifongique, d'antihistaminiques, d'antihypertenseurs, d'antihypotenseurs, d'un antiparasitaire, d'un antivirus, d'inhibiteurs de récepteurs de surface cellulaire, du glucose, de glycosides cardiaques, d'hormones, d'immunothérapies, d'acides nucléiques et analogues, de protéines et de glycoprotéines, de sédatifs et d'un analgésique, de stéroïdes, de tranquilisants, de vaccins et d'eau.

7. Procédé de production d'une vésicule ou d'un liposome lipidique hétérovésiculaire possédant au moins deux substances différentes biologiquement actives, encapsulées séparément dans ses cavités aqueuses, comprenant les étapes consistant à :

   (a) fournir un premier composant lipidique dissous dans un ou plusieurs solvants organiques et ajouter dans ledit composant liquide un premier composant aqueux non miscible contenant une première substance biologiquement active à encapsuler,

   (b) former une première émulsion eau-dans-huile à partir des deux premiers composants non miscibles,

   (c) fournir un deuxième composant lipidique dissous dans un ou plusieurs solvants organiques et ajouter dans ledit composant lipidique un deuxième composant aqueux non miscible contenant une deuxième substance à encapsuler,

   (d) former une deuxième émulsion eau-dans-huile à partir des deux deuxièmes composants non miscibles,

   (e) former une émulsion chimère par association de la première émulsion eau-dans-huile et de la deuxième émulsion eau-dans-huile,

   (f) transférer et immerger le produit de l'étape (e) dans un troisième milieu qui est non miscible avec lesdits solvants organiques,

   (g) disperser l'émulsion chimère pour former des sphérules de solvants contenant de nombreuses gouttelettes du premier composant aqueux contenant la première substance et du deuxième composant aqueux contenant la deuxième substance et

   (h) évaporer les solvants organiques des sphérules de solvants pour former les liposomes hétérovésiculaires.

8. Procédé selon la revendication 7, dans lequel on associe trois émulsions eau-dans-huile ou plus contenant trois composants aqueux non miscibles ou plus pour former l'émulsion chimère.

9. Procédé selon la revendication 7 ou la revendication 8, dans lequel les premier et deuxième composants lipidiques sont identiques.

10. Procédé selon l'une quelconque des revendications 7 à 9, dans lequel un ou plusieurs composants lipidiques contiennent un lipide avec une charge ou des charges globales négatives.

**11.** Procédé selon l'une quelconque des revendications 7 à 9, dans lequel au moins un composant parmi les premier et deuxième composants lipidiques est un lipide neutre soit seul, soit en association avec une substance choisie dans le groupe constitué de triglycérides, d'huiles végétales, de graisses animales, de tocophérols, d'esters de tocophérol, d'esters de chlolestérol et d'hydrocarbures.

**12.** Procédé selon l'une quelconque des revendications 7 à 11, dans lequel les premier et deuxième composants lipidiques sont un phospholipide ou un mélange de plusieurs phospholipides.

**13.** Procédé selon la revendication 12, dans lequel on fournit au moins l'un des phospholipides mélangé avec du cholestérol.

**14.** Procédé selon la revendication 12 ou la revendication 13, dans lequel on fournit au moins l'un des phospholipides mélangé avec de la stéarylamine.

**15.** Procédé selon l'une quelconque des revendications 12 à 14, dans lequel on choisit les phospholipides dans le groupe constitué de la phosphatidylcholine, de la cardiolipine, de la phosphatidyléthanolamine, de la sphingomyéline, de la lysophosphatidylcholine, de la phosphatidylsérine, du phosphatidylinositol, du phosphatidylglycérol et de l'acide phosphatidique.

**16.** Procédé selon l'une quelconque des revendications 7 à 15, dans lequel au moins une substance parmi les première et deuxième substances est un produit lipophile biologiquement actif.

**17.** Procédé selon l'une quelconque des revendications 7 à 15, dans lequel la substance biologiquement active est hydrophile.

**18.** Procédé selon la revendication 17, dans lequel on choisit la substance hydrophile biologiquement active dans le groupe constitué de l'interleukine 2, de la cytosine arabinoside, du méthotrexate, du 5-fluorouracile, de la cisplatine, de la floxuridine, du melphalan, de la mercaptopurine, de la thioguanine, du thiotépa, de la vincristine, de la vinblastine, de la streptozocine, du leuprolide, de l'interféron, de la calcitonine, de la doxorubicine, de la daunorubicine, du mitoxantrone, de la cellule amacrine, de l'actinomycine et de la bléomycine.

**19.** Procédé selon l'une quelconque des revendications 7 à 15, dans lequel on choisit les substances biologiquement actives dans le groupe constitué d'un antiarythmique, d'un antiasthmatique, d'un antibiotique, d'un anticancéreux, d'un antidiabétique, d'un antifongique, d'antihistaminiques, d'antihypertenseurs, d'antihypotenseurs, d'un antiparasitaire, d'un antivirus, d'inhibiteurs de récepteurs de surface cellulaire, du glucose, de glycosides cardiaques, d'hormones, d'immunothérapies, d'acides nucléiques et analogues, de protéines et de glycoprotéines, de sédatifs et un analgésique, de stéroïdes, de tranquilisants, de vaccins et de l'eau.

**20.** Procédé selon l'une quelconque des revendications 7 à 15, dans lequel on choisit les substances biologiquement actives à encapsuler dans le groupe constitué des compositions du tableau 1.

**21.** Procédé selon l'une quelconque des revendications 7 à 20, dans lequel on choisit le solvant organique dans le groupe constitué d'éthers, d'hydrocarbures, d'hydrocarbures halogénés, d'éthers halogénés, d'esters et de leurs associations.

**22.** Procédé selon l'une quelconque des revendications 7 à 21, dans lequel au moins l'une des substances biologiquement actives est encapsulée en présence d'un chlorhydrate.

**23.** Procédé selon la revendication 22, dans lequel on choisit le chlorhydrate dans le groupe constitué de l'acide chlorhydrique, du chlorhydrate de lysine, du chlorhydrate d'histidine et de leurs associations.

**24.** Procédé selon l'une quelconque des revendications 7 à 23, dans lequel on met en oeuvre l'émulsification des deux composants en utilisant des procédés choisis dans le groupe constitué d'une agitation mécanique, d'une énergie par ultrasons et d'une pulvérisation par une buse.

**25.** Procédé selon l'une quelconque des revendications 7 à 24, dans lequel le troisième composant aqueux contient au moins un agent de neutralisation d'acide.

**26.** Procédé selon la revendication 25, dans lequel on choisit l'agent de neutralisation d'acide, soit seul, soit en association, dans le groupe constitué de la lysine base libre et de l'histidine base libre.

**27.** Procédé selon la revendication 25 ou la revendication 26, dans lequel le troisième composant aqueux est une solution aqueuse contenant de plus des solutés choisis dans le groupe constitué des glucides et des acides aminés.

**28.** Procédé selon la revendication 27, dans lequel on choisit les solutés, soit seuls, soit en association, dans le groupe constitué du glucose, du saccharose, du lactose, de la lysine base libre et de l'histidine base libre.

**29.** Procédé selon l'une quelconque des revendications 7 à 28, dans lequel on met en oeuvre la dispersion pour former des sphérules de solvants en utilisant des procédés choisis dans le groupe constitué d'une agitation mécanique, d'une énergie par ultrasons et d'une pulvérisation par une buse.

**30.** Procédé selon l'une quelconque des revendications 7 à 29, dans lequel on réalise l'évaporation du solvant organique en faisant passer de l'azote gazeux sur le deuxième composant.

FIG. 8

FIG. 7

FIG. 6

FIG. 5

FIG. 4

FIG. 2

FIG. 3

FIG. 1